# EUROPEAN PATENT SPECIFICATION

(11) **EP 0 434 856 B1**
(45) Date of publication and mention of the grant of the patent: **13.07.1994**
(21) Application number: 89123903.0
(22) Date of filing: 23.12.1989
(51) Int. Cl.: G06F 15/20, A61B 5/08

(54) **Method of deriving a respiration signal and/or a cardiac artifact signal from a physiological signal**
Verfahren zur Gewinnung von einem Atmungssignal und/oder einem Herzstörsignal von einem physiologischen Signal
Méthode pour l'obtention d'un signal respiratoire et/ou d'un signal cardiaque parasite à partir d'un signal physiologique

(43) Date of publication of application: 03.07.1991
(73) Proprietor: Hewlett-Packard GmbH, D-71004 Böblingen (DE)
(72) Inventor: Reiche, Martin, Dipl.-Ing., D-7046 Gäufelden 2 (DE)
(74) Representative: Kurz, Peter

(56) References cited:
- US-A- 4 422 458
- US-A- 4 582 068
- US-A- 4 781 201

## Description

The present invention relates to a method of deriving a respiration signal and/or a cardiac artifact signal from a physiological signal having at least a respiration signal component and a cardiac artifact signal component, in particular from an impedance pneumography signal, according to the generic clause of patent claim 1.

The present invention deals in particular with a method of deriving or producing a respiration signal and/or a cardiac artifact signal on the basis of an impedance pneumography signal detected by means of electrodes placed on the thorax of a patient. Impedance pneumography usually serves for monitoring the respiratory activity of spontaneously breathing patients and employs for signal detection the electrical impedance of the thorax which is influenced by the patient's respiratory activity and, thus, changes as a function of time.

It is generally known that an impedance pneumography signal does not only have a respiration signal component, but has additional artifacts superimposed thereon which particularly include a cardiac artifact signal component which designates hereinafter the impedance change occurring during the blood flow associated with the systole. Additional parameters disturbing the impedance pneumography signal are, for example, spontaneous motion artifacts of the patient. While motion artifacts can hardly be eliminated, there are already a number of methods having the aim of filtering out the cardiac artifact signal component from an impedance pneumography signal in order to obtain a respiration signal. US-A-4422458 discloses a method for detecting respiratory distress wherein the increasing force of contraction of the patient's heart which effects the impedance signal may be detected by utilizing a heartbeat signal to generate a trigger pulse. The heartbeat moment is detected by a cardiac monitor generating the output trigger pulse. Samples of the impedance signal are obtained at each instant a trigger pulse is generated. These samples are stored and compared to previously obtained samples. In case a steadily increasing trend of the samples is detected, a sound alarm signalling respiratory distress condition is generated. Thus, this prior art reference does not relate to a filtering technique for obtaining a mere respiration signal and/or a mere cardiac artifact signal from the impedance pneumography signal.

US-A-4781201 discloses an adaptive filtering system for suppressing the cardiac artifact component from the impedance pneumography signal. An adaptive filtering system varies the filter characteristics such that portions of the respiration signal having a frequency content at or above the heart rate are most greatly attenuated. This prior art system produces good filtering results only in the case of a good periodicity of the cardiac artifact signal component to be filtered out.

For example, EP-B1-0082655 just as US-C-4,537,196 and US-C-4,582,068 - the latter two being the priority basis of the first-mentioned European Patent -, discloses a method of processing a physiological signal, which in particular may be an impedance pneumography signal, wherein the cardiac artifact signal masking the respiration signal is assumed to be periodical so that, for calculation of a filtered signal, the artifact frequency assumed to be constant is ascertained at first and thereafter a signal of the particular frequency in the impedance pneumography signal on the input side is suppressed in a computer filter so as to produce the filtered signal. The known method necessarily requires a threshold detection circuit for determining a threshold value as a function of preceding levels of previously filtered impedance pneumography signals, with said threshold detection circuit being fed with the filtered impedance pneumography signal in order to produce a periodic repetition signal indicating recurrence of the respiration signal when the filtered functional signal exceeds the threshold value of the threshold detection circuit. This known filtering technique is capable of suppressing only the basic component of a cardiovascular artifact occurring with a fixed periodicity. However, since even the requirement of maintaining a periodicity of the disturbing signal is not fulfilled in practical life, the filtering results obtainable by the known method fail to be satisfactory.

EP-A2-0048591 discloses a further filtering technique for obtaining a respiration signal from an impedance pneumography signal by filtering out a cardiac masking. In case of this known method the heart rate is measured by means of an electrocardiograph for determining the heartbeat period which is used for delaying the impedance signal on the input side by one heartbeat period and for subtracting this signal therafter from the undelayed impedance signal. This known filtering technique also necessarily operates in insufficient manner when the precondition of the periodicity of the cardiovascular artifact is not fulfilled or not fulfilled with sufficient accuracy.

WO89/01312 discloses a method of processing impedance signals which serves for filtering out a respiration signal component from the signal representing the impedance of the thorax. For suppressing the stronger impedance change of the thorax due to respiration as compared to the impedance change due to heart activity, a clamping circuit is activated in synchronization with the heart activity at a moment of time before the beginning of the mechanical systole. This circuit is only opened during the duration of the mechanical systole so that only the voltage fluctuation constitutes the output signal, in essence on the basis of the mechnical systole. However, according to nature, the cardiovascular signal is masked by a slight respiration signal component during this period of time as well, thus making this filtering technique unsuitable for obtaining high accuracy.

US-C-3,976,052 reveals a method of deriving a respiration signal from an impedance pneumography signal by filtering out a cardiac artifact signal, in which the heart rate is measured and the period of the heartbeat is compared to the period of the impedance pneumography signals. When the durations of the periods of both signals are essentially identical, i.e. when the impedance pneumography signals measured essentially are caused by the heartbeat, a carrier threshold value is increased to such an extent that further response to signals caused by heartbeat is prevented whereby, after this automatic basic adjustment, respiration signals having an amplitude usually in excess of the amplitude of cardiac artifact signals are automatically detected.

US-C-3,608,542 reveals a further filtering method for impedance pneumography in which, for suppressing cardiovascular signal components, adjustable narrow-band filters and adjustable level detectors are employed so that this known method can also produce good filtering results only with good periodicity of the cardiac artifact signal component to be filtered out.

With respect to this prior art, it is the object of the present invention to develop a method of deriving a respiration signal and/or a cardiac artifact signal from a physiological signal having at least a respiration signal component and a cardiac artifact signal component, in such a manner that higher accuracy of the respiration signal and/or the cardiac artifact signal derived from the impedance pneumography signal is obtained.

With the method according to the generic clause of patent claim 1, this object is met by the method steps indicated in the characterizing clause of patent claim 1.

The invention starts from the realization that the cardiac artifact signal can by no means be assumed to be a signal having a fixed period or a period changing only slowly in time, but that it is a signal which, starting from a specific starting point in terms of time within the heartbeat cycle - designated in the following as heartbeat moment -, has a signal pattern or waveform which in terms of time remains substantially the same from one heartbeat to another heartbeat. The invention simulates this signal pattern by means of an adaptive or learning signal or learning function in a self-adaptive routine in that the heartbeat moment is at first detected and the amplitude of the physiological signal at this moment is stored as a reference amplitude. Thereafter, the learning signal having a pattern related in terms of time of the heartbeat moment is calculated and stored on the basis of the difference between the instantaneous amplitude of the physiological signal and the stored amplitude thereof as well as on the basis of previous learning signals. With a suitable selection of the calculation of the learning signals on the basis of said difference and the previous learning signals, components of the physiological signal not correlated with the heatbeat moment are reduced to average whereas components correlated therewith increase so that the learning signal continuously adapts itself to the current cardiac artifact signal component. As is elucidated in more detail with reference to the dependent claims, the learning signal obtained in this way may be used on the one hand directly as an output signal when the cardiac artifact signal is desired as an output signal, or can be employed on the other hand, by subtraction from the physiological signal, for calculating the respiration signal freed from the cardiac artifact signal.

The method according to claim 2 allows derivation of the respiration signal and/or cardiac artifact signal by means of a digital circuit, in particular by means of a commerically available microcomputer. The learning signal for this purpose is divided into a plurality of learning signal values so that it can be stored in a semi-conductor memory under a number of addresses corresponding to time increments of the learning signal starting from the heartbeat moment.

When the method according to the invention is to be employed for deriving or calculating a respiration signal on the basis of the impedance pneumography signal, the respiration signal can be freed from the cardiac artifact signal components by subtracting the learning signal having a pattern related in terms of time to each heartbeat moment from the impedance pneumography signal.

The development of the method according to claim 3, as defined in claim 4, is particularly well suited for being carried out by means of digital circuit, especially a commerically available microcomputer in which the time value is reset upon occurrence of each heartbeat moment and is then incremented so that it may be used as an access address for reading out as well as for storing the particular learning signal values of the learning signal.

As defined in claim 5, the particular new learning signal values can be derived by means of a simple calculation rule from the former learning signal values for corresponding distances in time from the cardiac artifact signal moment and the difference between the instantaneous amplitude and the stored amplitude of the physiological signal. By weighting of the former learning signal value and the difference mentioned, a learning function is achieved which results in a learning signal simulating the cardiac artifact signal, with the cardiac artifact signal detected last contributing most to said learning signal and the cardiac artifact signals detected therebefore contributing thereto to a lesser and lesser degree as the distance in time increases. This learning function results in continuous adaptation of the learning signal to the cardiac artifact signal, eliminates signal components not related to the heartbeat moment by time averaging and implements also an increasing "oblivion" of earlier contributions of cardiac artifact signals which are further back in the course of time.

As defined in claim 6, the heartbeat moment can be derived preferably from an electrocardiograph through the pulse of a QRS signal. This way of determining the heartbeat moment proves to be expedient since the QRS signal is a parameter that is already present in usual patient monitors, thus the same may be utilized. It is to be noted that the term "heartbeat moment" in the sense of claim 6 may be any reference point within a cardiac artifact signal.

Special importance is attached to the amplitude limitation of the learning signal according to claims 7 and 8. The subject-matter of these claims is based on the realization that cardiovascular artifacts or cardiac artifact signals have a limited amplitude whose maximum value is typically less than 0,5 ohm. Also in case of weak respiration, the respiration signal amplitude is typically in the order of magnitude of 1 ohm. During impedance pneumography monitoring of newborn babies the extreme situation may occur that the respiratory rate is identical to the heart rate. Due to amplitude limitation of the learning signal, it is also prevented in this case that the respiration signals are made zero by erroneous integration thereof in the learning signal due to the indentical rates of said signals, so that safe respiration signal detection is ensured for this extreme case as well.

In accordance with claim 9, the cardiac artifact signal can be simulated by reading out the learning signal in synchronization with the detection of the particular heartbeat moment.

In the following a preferred embodiment of an apparatus according to the invention, operating in accordance with the inventional method of deriving a respiration signal and/or a cardiac artifact signal from an impedance pneumography signal, will be elucidated with reference to the accompanying drawings in which
- Fig. 1: shows an apparatus for deriving a respiration signal from an impedance pneumography signal and a QRS signal;
- Fig. 2: shows a flow diagram of the method of the invention, illustrating the operation of the microcomputer of the apparatus of the invention according to Fig. 1;
- Figs. 3a, 3b, 4a, 4b: show exemplary impedance pneumography signals on the input side as well as respiration signals which are largely freed from cardiac artifact signal components and calculated from said impedance pneumography signals by means of the method according to the invention.

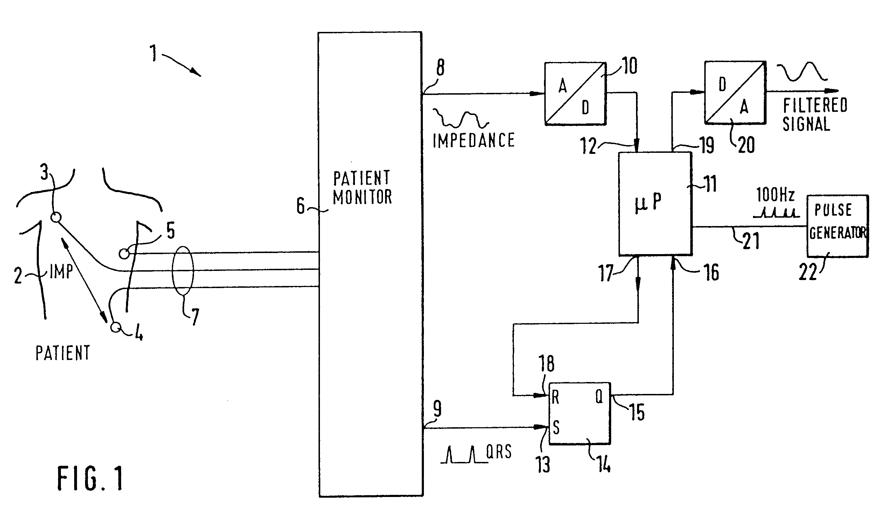

As illustrated in Fig. 1, an apparatus according to the invention which is suitable for carrying out the method according to the invention and which, as a whole, is designated with the reference numeral 1, comprises two impedance measuring electrodes placed on the thorax of a patient for determining the thorax impedance present therebetween, in order to derive therefrom an impedance pneumography signal as well as an ECG electrode 5 for producing an electrocardiography signal. The signals mentioned are supplied to a patient monitor 6 via a three-wire electrode-patient cable 7. The patient monitor is preferably a monitor of the type "Hewlett-Packard 78354".

The monitor produces on its output side an impedance pneumography signal on a first output 8 as well as a QRS signal, which is representative of the heartbeat moment and derived from the ECG signal on a second output 9.

The apparatus 1 to 8 described so far for obtaining the impedance pneumography signal and the QRS signal is known as such from the prior art and, therefore, need not be elucidated in detail.

The impedance pneumography signal from the first output 8 is converted by an A/D converter 10 to a digital signal that is fed to a microcomputer 11 on the first input port 12 thereof. The pulse-like QRS signal from the second output 9 of the patient monitor 6 is fed to the setting input 13 of an RS-flip-flop 14. A Q-output 15 of RS-flip-flop 14 is connected to a second input port 16 of microcomputer 11. As soon as the microcomputer 11 has detected the presence of a high logical level at its second input port 16, which is caused by a preceding QRS pulse, the microcomputer 11 generates at its first output 17 a resetting signal which is fed to the resetting input 18 of the RS-flip-flop 14. As is evident for the expert, the RS-flip-flop 14 serves for ensuring safe detection of a pulse of the QRS signal by the microcomputer 11. The filtered respiration signal is produced by the microcomputer at a second output port 19 connected to a D/A converter 20.

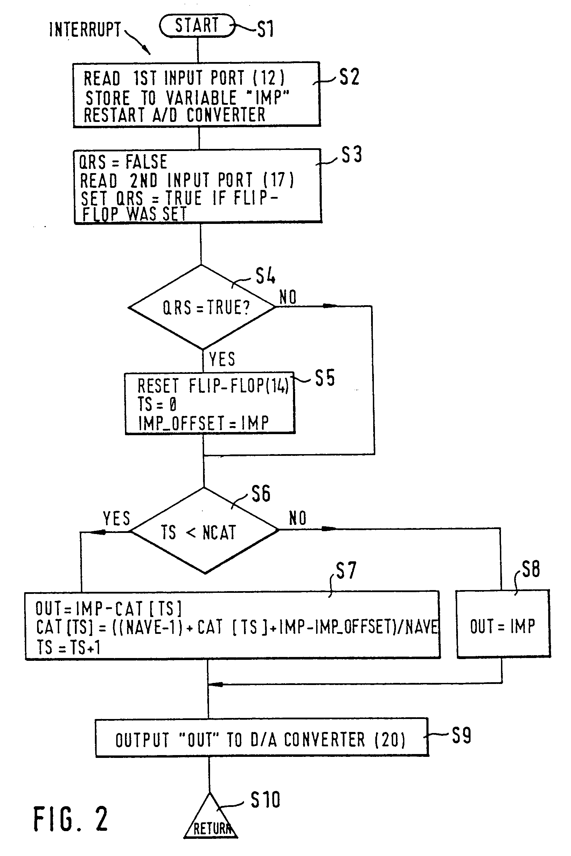

A pulse generator 22 feeds its waveform into the interrupt line 21 of processor 11 thus enforcing the continuous repetition of the execution of the program outlined below (Fig. 2). The preferred execution frequency is about 100 Hz.

In the following, a flow diagram of the program will be elucidated with reference to Fig. 2, indicating the manner in which the microcomputer operates for carrying out the inventional method of calculating a filtered respiration signal from an impedance pneumography signal.

Subsequent to an interrupt signal (21, Fig. 1) at steps S1, the micrcomputer 11 reads the impedance value present at its first input port 12, stores the same as a variable "IMP" and effects restarting of the A/D converter 10 in a second program step S2.

In a third program step S3 the logical value QRS, which is representative of the presence or absence of a QRS pulse, is initialized to the value "FALSE". The logical value issued from the output of flip-flop 14 and present at the second input port 16 is now read in. As soon as this logical value QRS is true and the flip-flop is thus set, the logical value QRS is set to "TRUE".

It is examined in a fourth program step S4 whether the logical value QRS is "TRUE". If this is so, the program continues with a fifth program step S5. Otherwise, the programs jumps to a sixth program step S6.

In the fifth program step the flip-flop 14 is reset in that a logic high level is produced at the first output 17 of the microcomputer, and a value TS representing the time axis is set to zero. The amplitude value IMP of the impedance pneumography signal present at this reference time is stored as a reference amplitude IMP OFFSET.

In the sixth program step S6 it is examined whether the instantaneous time value TS is smaller than a maximum value NCAT representative of the maximum duration of the learning signal starting from the occurrence of the heartbeat moment (TS = 0). If this is so, the program proceeds with a seventh program step S7, whereas it otherwise continues with an eighth program step S8.

In the seventh program step S7 the output signal OUT of the microcomputer is first set to the difference between the instantaneous impedance value IMP and the learning signal value CAT(TS) associated with the instantaneous time value TS.

In a subsequent step a new learning signal value CAT(TS) is set by multiplying the former learning signal value CAT(TS) by an averaging constant NAVE reduced by 1, and this value is increased by the difference between the instantaneous amplitude and the stored reference amplitude (IMP - IMP OFFSET) and the resulting quantity is divided by the averaging constant NAVE.

Thereafter the instantaneous time value TS is incremented by 1.

In case the result of the examination in the sixth program step S6 is negative, the output value OUT is set in the eighth program step S8 to the instantaneous value of the impedance pneumography signal IMP since in this case no calculatory correction thereof has to be carried out.

In a ninth program step S9 the output value OUT calculated in this manner is supplied to the D/A converter 20 as a filtered output signal which is issued by said converter in analog form.

After step S9, the processor waits for a new interrupt at step S10 before returning to the fourth programm step S4.

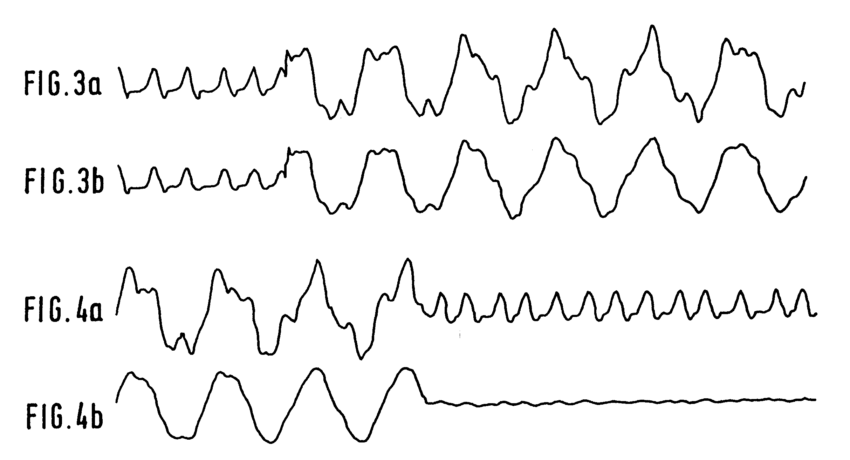

Figs. 3a and 4a (continuation) shows a pattern of a pneumography signal which was artificially simulated. Figs. 3b and 4b the show output signal curve calculated in accordance with the method of the invention, with the afore-described routined having been used for the calculation. For a better understanding of the curves, it is to be noted that the learning signal values "CAT(TS)" were set to zero at the beginning of the particular calculation routines. As the learning process proceeds, which can be observed on the basis of the output curves of Figs. 3b, 4b, the disturbing component in the input signal, which corresponds to the cardiac artifact signal component, is suppressed in increasing manner such that the filtered signal corresponds to a pure respiration signal.

The operability of the method according to the invention could also be proved upon occurrence of ventricular extrasystoles at an irregular heart rate and in case of erroneous QRS determinations.

Although the method according to the invention, in the embodiment elucidated, is used only for obtaining a respiration signal freed from cardiac artifact signal components on the basis of an impedance pneumography signal, it may also be used for producing a cardiac artifact signal freed from respiration signal components on the basis of an impedance pneumography signal, or for producing both signals simultaneously.

## Claims

1. A method of deriving a respiration signal and/or a cardiac artifact signal from a physiological signal having at least a respiration signal component and a cardiac artifact signal component, in particular from an impedance pneumography signal, comprising the steps of:
- detecting the heartbeat moment (S3, S4), and
- storing the amplitude of the physiological signal at the heartbeat moment (S5),
characterized by the step of:
- calculating and storing a learning signal (CAT(TS)) representing the cardiac artifact signal having a pattern related in terms of time to the heartbeat moment (TS), on the basis of the difference between the instantaneous amplitude of the physiological signal (IMP) and the stored amplitude (IMP OFFSET) thereon and on the basis of a previous learning signal (CAT(TS), S7).

2. A method according to claim 1,
characterized in that method step (S7) of calculating and storing the learning signal (CAT(TS)) comprises calculating and storing a plurality of learning signal values (CAT(1) to CAT(NCAT - 1)) for a plurality of moments of time each having a predetermined time distance from the heartbeat moment, on the basis of the particular difference between the instantaneous amplitude of the physiological signal and the stored amplitude of the physiological signal as well as on the basis of the previous learning signal value.

3. A method according to claim 1 or 2,
characterized in that, for deriving a respiration signal freed from cardiac artifact signal components or cardiovascular artifacts, the learning signal having a pattern related in terms of time to each heartbeat moment is subtracted form the impedance pneumography signal.

4. A method according to claim 3 when dependent upon claim 2,
characterized in that for each heartbeat cycle starting with a heartbeat moment, a time value (TS) is incremented starting from each heartbeat moment and used for having access to the learning signal value (CAT(TS)) corresponding to the particular distance in time (TS) from the heartbeat moment (QRS = TRUE), such that said learning signal value is subtracted during this heartbeat cycle from the instantaneous impedance pneumography signal until access is had to the learning signal value (CAT(TS+1)) associated with the subsequent distance in time of this heartbeat cycle.

5. A method according to any of claims 1 to 4,
characterized in that the new learning signal value associated with a specific distance in time to the heartbeat moment is calculated from the former learning signal value associated with this distance in time and from the instantaneous difference between the instantaneous amplitude and the stored amplitude of the physiological signal, by multiplying this former learning signal value by a factor smaller than 1 ((NAVE - 1)/NAVE) and by adding to the resulting product the product of the difference of the instantaneous and the stored amplitude on the one hand (IMP - IMP OFFSET) and the difference from 1 reduced by this factor on the other hand (1/NAVE).

6. A method according to claim 5,
characterized in that the heartbeat moment is derived from an electrocardiograph through the pulse of a QRS signal.

7. A method according to any of claims 1 to 6,
characterized in that the learning signal amplitude or learning signal value (CAT(TS)) is limited to a maximum value which is lower than usual respiration signal amplitudes.

8. A method according to claim 7,
characterized in that the physiological signal is an impedance pneumography signal, and that the maximum value of the learning signal amplitude or maximum learning signal value is limited to 1 ohm at the most.

9. A method according to any of claims 1 to 8,
characterized in that, for deriving a cardiac artifact signal freed from respiration signal components, the learning signal is read out periodically starting with each heartbeat moment.

## Patentansprüche

1. Ein verfahren zum Ableiten eines Atmungssignales und/oder eines Herzartefaktsignales aus einem zumindest einen Atmungssignalanteil und einen Herzartefaktsignalanteil aufweisenden physiologischen Signal, insbesondere einem Impedanzpneumatographiesignal,
mit folgenden Schritten:
- Erfassen des Herzschlagzeitpunktes (S3, S4), und
- Speichern der Amplitude des physiologischen Signales zum Herzschlagzeitpunkt (S5),
gekennzeichnet durch folgenden Schritt:
- Berechnen und Speichern eines Lernsignales (CAT(TS)) mit einem auf den Herzschlagzeitpunkt (TS) bezogenen zeitlichen Verlauf aufgrund der Differenz zwischen der momentanen Amplitude des physiologischen Signales (IMP) und der gespeicherten Amplitude (IMP OFFSET) desselben sowie aufgrund eines vorherigen Lernsignales (CAT(TS), S7).

2. Ein verfahren nach Anspruch 1,
dadurch gekennzeichnet, daß der Verfahrensschritt (S7) des Berechnens und Speicherns des Lernsignales (CAT(TS)) das Berechnen und Speichern einer Mehrzahl von Lernsignalwerten (CAT(1) bis CAT(NCAT - 1)) für eine Mehrzahl von Zeitpunkten mit jeweils vorgegebenen zeitlichen Abständen von dem Herzschlagzeitpunkt aufgrund der jeweiligen Differenz zwischen der momentanen Amplitude des physiologischen Signales und der gespeicherten Amplitude des physiologischen Signales sowie aufgrund eines vorherigen Lernsignalwertes umfaßt.

3. Ein Verfahren nach Anspruch 1 oder 2,
dadurch gekennzeichnet, daß zum Ableiten eines von Herzartefaktsignalanteilen oder cardiovaskularen Artefakten befreiten Atmungssignales das Lernsignal jeweils mit einem auf jeden Herzschlagzeitpunkt bezogenen zeitlichen Verlauf von dem Impedanzpneumatographiesignal subtrahiert wird.

4. Ein Verfahren nach Anspruch 3 in Rückbeziehung auf Anspruch 2,
dadurch gekennzeichnet, daß für jeden bei einem Herzschlagzeitpunkt beginnenden Herzschlagzyklus jeweils beginnend ab jedem Herzschlagzeitpunkt ein Zeitwert (TS) inkrementiert wird, mit dem auf den dem jeweiligen zeitlichen Abstand (TS) von dem Herzschlagzeitpunkt (QRS = TRUE) entsprechenden Lernsignalwert (CAT(TS)) zugegriffen wird, so daß dieser während dieses Herzschlagzyklus so lange von dem momentanen Impedanzpneumatographiesignal subtrahiert wird, bis auf den dem nachfolgenden zeitlichen Abstand dieses Herzschlagzyklus zugeordneten Lernsignalwert (CAT(TS+1)) zugegriffen wird.

5. Ein Verfahren nach einem der Ansprüche 1 bis 4,
dadurch gekennzeichnet, daß der einem bestimmten zeitlichen Abstand zu dem Herzschlagzeitpunkt zugeordnete neue Lernsignalwert aus dem diesem zeitlichen Abstand zugeordneten bisherigen Lernsignalwert und der momentanen Differenz zwischen der momentanen Amplitude und der gespeicherten Amplitude des physiologischen Signales dadurch berechnet wird, daß dieser bisherige Lernsignalwert mit einem Faktor, der kleiner als 1 ist ((NAVE - 1) / NAVE) multipliziert wird und zu dem sich ergebenden Produkt das Produkt aus der Differenz der momentanen und der gespeicherten Amplitude einerseits (IMP - IMP OFFSET) und der Differenz aus 1 vermindert um diesen Faktor andererseits (1 / NAVE) addiert wird.

6. Ein Verfahren nach Anspruch 5,
dadurch gekennzeichnet, daß der Herzschlagzeitpunkt durch den Puls eines QRS-Signales von einem Elektrokardiographen abgeleitet wird.

7. Ein Verfahren nach einem der Ansprüche 1 bis 6,
dadurch gekennzeichnet, daß die Lernsignalamplitude oder der Lernsignalwert (CAT(TS)) auf einen Maximalwert, der unterhalb von üblichen Atemsignalamplituden liegt, begrenzt ist.

8. Ein Verfahren nach Anspruch 7,
dadurch gekennzeichnet, daß das physiologische Signal ein Impedanzpneumatographiesignal ist, und
daß der Maximalwert der Lernsignalamplitude bzw. der maximale Lernsignalwert auf maximal 1 Ohm begrenzt ist.

9. Ein Verfahren nach einem der Ansprüche 1 bis 8,
dadurch gekennzeichnet, daß zum Ableiten eines von Atmungssignalanteilen befreiten Herzartefaktsignales das Lernsignal periodisch beginnend mit jedem Herzschlagzeitpunkt ausgelesen wird.

## Revendications

1. Un procédé de dérivation d'un signal de respiration et/ou d'un signal cardiaque parasite à partir d'un signal physiologique comprenant au moins une composante de signal de respiration et une composante de signal cardiaque parasite, en particulier à partir d'un signal de pneumographie d'impédance, comprenant les étapes consistant à:
- détecter l'instant d'un battement de coeur (S3, S4), et
- mémoriser l'amplitude du signal physiologique à l'instant du battement de coeur (S5),
caractérisé par l'étape consistant à
calculer et mémoriser un signal d'apprentissage (CAT(TS)) représentant le signal cardiaque parasite dont la configuration est liée en termes de temps au moment du battement de coeur (TS), sur la base de la différence entre l'amplitude instantanée du signal physiologique (IMP) et l'amplitude mémorisée (IMP OFFSET) de celui-ci et sur la base du signal d'apprentissage précédent (CAT(TS), S7).

2. Un procédé selon la revendication 1,
caractérisé en ce que l'étape (S7) du procédé qui consiste à calculer et mémoriser le signal d'apprentissage (CAT(TS)) comprend l'étape consistant à calculer et mémoriser une série de valeurs du signal d'apprentissage (CAT(1) à CAT(NCAT - 1)) pour une série d'instants qui sont situés chacun à une distance temporelle prédéterminée de l'instant du battement de coeur, sur la base de la différence particulière entre l'amplitude instantanée du signal physiologique et l'amplitude mémorisée du signal physiologique, ainsi que sur la base de la valeur du signal d'apprentissage précédent.

3. Un procédé selon la revendication 1 ou 2,
caractérisé en ce que, le signal d'apprentissage dont la configuration est liée en terme de temps à chaque instant de battement de coeur est soustrait du signal de pneumographie d'impédance afin de dériver un signal de respiration exempt de composantes du signal cardiaque parasite ou de parasites cardio-vasculaires.

4. Un procédé selon la revendication 3 lorsqu'elle dépend de la revendication 2,
caractérisé en ce que, pour chaque cycle de battement de coeur commençant à un instant de battement de coeur, une valeur temporelle (TS) est incrémentée en commençant à chaque instant de battement de coeur et est utilisée afin d'accéder à la valeur du signal d'apprentissage (CAT(TS)) correspondant à la distance temporelle particulière (TS) à partir de l'instant du battement de coeur (QRS = TRUE), d'une manière telle que ladite valeur de signal d'apprentissage soit soustraite pendant ce cycle de battement de coeur du signal instantané de pneumographie d'impédance jusqu'à ce qu'un accès soit obtenu à la valeur du signal d'apprentissage (CAT(TS + 1)) associé à la distance temporelle ultérieure de ce cycle de battement de coeur.

5. Un procédé selon l'une quelconque des revendications 1 à 4,
caractérisé en ce que la nouvelle valeur de signal d'apprentissage, associée à une distance temporelle spécifique vis-à-vis de l'instant du battement de coeur, est calculée à partir de la valeur précédente du signal d'apprentissage associée à cette distance temporelle et à partir de la différence instantanée entre l'amplitude instantanée et l'amplitude mémorisée du signal physiologique, en multipliant cette valeur précédente du signal d'apprentissage par un facteur inférieur à 1 ((NAVE - 1)/NAVE) et en ajoutant au produit résultant le produit de la différence entre l'amplitude instantanée et l'amplitude mémorisée (IMP - IMP OFFSET), d'une part, par la différence par rapport à 1 réduite par ce facteur (1/NAVE), d'autre part.

6. Un procédé selon la revendication 5,
caractérisé en ce que l'instant de battement de coeur est dérivé d'un électrocardiographe à l'aide de l'impulsion d'un signal QRS.

7. Un procédé selon l'une quelconque des revendications 1 à 6,
caractérisé en ce que l'amplitude du signal d'apprentissage ou valeur du signal d'apprentissage (CAT(TS)) est limitée à une valeur maximale qui est inférieure aux amplitudes habituelles du signal de respiration.

8. Un procédé selon la revendication 7,
caractérisé en ce que le signal physiologique est un signal de pneumographie d'impédance, et
que la valeur maximale de l'amplitude du signal d'apprentissage ou valeur maximale du signal d'apprentissage est limitée à 1 ohm au plus.

9. Un procédé selon l'une quelconque des revendications 1 à 8, caractérisé en ce que le signal d'apprentissage est lu périodiquement en commençant à chaque instant de battement de coeur de manière à dériver un signal cardiaque parasite exempt des composantes de signal de respiration.
